# EUROPÄISCHE PATENTSCHRIFT

(11) **EP 0 907 900 B1**
(45) Veröffentlichungstag und Bekanntmachung des Hinweises auf die Patenterteilung: **10.09.2003**
(21) Anmeldenummer: 98919177.0
(22) Anmeldetag: 27.03.1998
(51) Int. Cl.: G02B 23/24, A61B 1/00, G02B 7/04

(54) **VORRICHTUNG ZUM POSITIONIEREN VON BAUELEMENTEN INNERHALB ENDOSKOPISCHER SYSTEME**
DEVICE FOR POSITIONING COMPONENTS IN ENDOSCOPIC SYSTEMS
DISPOSITIF POUR LE POSITIONNEMENT D'ELEMENTS A L'INTERIEUR DE SYSTEMES ENDOSCOPIQUES

(30) Priorität: 29.03.1997 DE 19713276
(43) Veröffentlichungstag der Anmeldung: 14.04.1999
(60) Teilanmeldung: 02026641.7
(73) Patentinhaber: Karl Storz GmbH & Co. KG, 78532 Tuttlingen (DE)
(72) Erfinder: KEHR, Ulrich, D-73760 Ostfildern (DE); RUDISCHHAUSER, Jürgen, D-78532 Tuttlingen (DE); DAHMEN, Jan, D-78606 Seitingen (DE)
(74) Vertreter: Heuckeroth, Volker, Dr.
(86) Internationale Anmeldenummer: EP9801825
(87) Internationale Veröffentlichungsnummer: WO98044376

(56) Entgegenhaltungen:
- EP-A- 0 845 694
- DE-A- 19 521 654
- DE-C- 970 298
- DE-U- 8 810 044
- US-A- 5 139 383
- US-A- 5 359 992
- US-A- 5 539 266

## Beschreibung

Die Erfindung betrifft eine Vorrichtung zum Positionieren von Bauelementen innerhalb endoskopischer Systeme, mit einem hermetisch dichten Gehäuse, mit einem um die Außenseite des Gehäuses drehbar angeordneten äußeren Ringelement, das umfänglich zumindest einen Magnet trägt, mit einem weiteren innerhalb des dichten Gehäuses angeordneten inneren Ringelement, das ebenfalls zumindest einen Magnet trägt, wobei die Magnete so ausgerichtet sind, daß eine Drehbewegung des äußeren Ringelementes über magnetischen Kraftschluß den zumindest einen inneren Magneten bewegt, wobei diese Bewegung zum Positionieren von Bauelementen dient.

Eine derartige Vorrichtung ist aus der DE 195 21 654 Al bekannt.

Positionieren im Sinne der vorliegenden Erfindung beinhaltet ein axiales Verschieben und/oder ein umfängliches Verschieben und dadurch in Stellung Bringen von Bauelementen.

Der Begriff Bauelemente umfaßt bspw. optische Bauelemente, z.B. Linsen in einem Optikkopf eines Endoskopes. Eine axiale Verschiebung der Systeme dient zum Fokussieren oder zum Scharfstellen des optischen Systems. Unter den Begriff Bauelemente fallen auch mechanische Bauelemente, die bspw. in ein optisches System ein- oder ausgeschwenkt werden sollen, wie bspw. Filter, Blenden oder dgl. Dabei kann die Handhabung der Positioniervorrichtung am proximalen Ende des endoskopischen Systems erfolgen, das zu bewegende Bauelement kann auch am distalen Ende angeordnet sein, und über ein Gestänge mit der Positioniervorrichtung verbunden sein.

Unter endoskopischen Systemen im Sinne der vorliegenden Erfindung werden Endoskope oder auch endoskopische Kamerasysteme verstanden.

Unter einem hermetisch dichten Gehäuse im Sinne der vorliegenden Erfindung versteht man ein derart abgeschlossenes Gehäuse, daß dieses bspw. autoklaviert werden kann, ohne daß die Gefahr besteht, daß bei den extremen Temperaturschwankungen in den Innenraum des Gehäuses Feuchtigkeit oder Flüssigkeiten, somit Kontaminationen eindringen können. Die Magnete des äußeren, um die Außenseite des dichten Gehäuses angeordneten Ringelements wirken mit den Magneten des innerhalb des dichten Gehäuses angeordneten Ringelements im Sinne einer Magnetkupplung.

Bei der eingangs genannten optischen Vorrichtung ist der innere Magnet in einer schraubenlinienförmigen Schlitzöffnung einer im Innern des dichten Gehäuses ortsfest angeordneten Hülse aufgenommen. Im Innern der Hülse ist axial verschiebbar und drehbar eine Fassung angeordnet, die Linsen einer Objektivgruppe trägt. Der als Rundmagnet ausgebildete innere Magnet greift in eine radiale Sacklochbohrung am Außenumfang der Fassung ein.

Der Magnet des äußeren drehbaren Ringelementes ist als Rechteckmagnet ausgebildet, der mit dem Rundmagneten in Wirkverbindung steht, also diesem in etwa gegenüberliegt.

Wird nunmehr das äußere Ringelement mit dem Rechteckmagneten gedreht, wird der innere Rundmagnet ebenfalls gedreht. Aufgrund der Tatsache, daß dieser in einem schraubenlinienförmigen Schlitz der Hülse aufgenommen ist, bewegt sich der Rundmagnet auch in axialer Richtung.

Demzufolge muß der am äußeren Ring angeordnete Rechteckmagnet eine solche axiale Erstreckung aufweisen, die dem maximalen axialen Laufweg des inneren Rundmagneten während dessen Lauf in dem schraubenlinienförmigen Schlitz in der Hülse entspricht.

Dadurch, daß der Rundmagnet in einer radialen Sacklochbohrung an der Außenseite der Fassung in Eingriff steht, wird diese entsprechend der axialen Vorschubbewegung des Rundmagneten axial verschoben.

Diese Ausgestaltung hat den Nachteil, daß aufgrund der axialen Beweglichkeit des inneren Magnetes der äußere Ringmagnet eine sehr lange axiale Erstreckung aufweisen muß, somit groß baut, was zu sperrigen und großen Bauweisen führt. Aufgrund der Tatsache, daß der Magnet im äußeren Ring ein relativ großer Magnet ist, streut dieser in Richtung Außenseite Magnetfelder aus, die Störungen an solchen Systemen ausüben können, die mit Elektronenstrahlungen arbeiten, also bspw. Monitore von endoskopischen Kameras oder dgl. Darüber hinaus steht der innere Rundmagnet mechanisch mit zwei unterschiedlichen Bauelementen in Verbindung, nämlich der äußeren ortsfesten Hülse mit dem schraubenlinienförmigen Schlitz und der Sacklochbohrung der inneren Fassung des optischen Systems. Um ein verklemmfreies Laufen zu gewährleisten, müssen exakt bearbeitete Teile und auch ein bestimmte Spiel vorhanden sein, so daß, insbesondere bei Umkehrbewegungen, ruckartige Bewegungen mit Verstellungen des optischen Systems verbunden sind.

Aus der US-A-5 359 992 ist eine Vorrichtung zum Positionieren von Bauelementen innerhalb endoskopischer Systeme bekannt, bei dem im äußeren Ringelement diametral gegenüberliegend zwei schraubenlinienförmige Schlitze ausgebildet sind, in denen jeweils diametral gegenüberliegend Rundmagnete eingesetzt sind. Die Rundmagnete greifen in eine axial verlaufende Aussparung an der Außenseite einer in dem Ring angeordneten Hülse an. Ein Drehen des äußeren Ringes verursacht somit eine Axialverschiebung des äußeren Magnetes. Im inneren abgeschlossenen Bereich sind entsprechend diametral gegenüberliegende Magnete vorhanden, die den Bewegungen des äußeren Magnetes folgen und somit die Kupplung bewirken. Das innere Stellelement mit den inneren Magneten ist mechanisch nicht geführt, so daß, falls das innere Element aufgrund eines Schockes oder eines Stoßes aus dem magnetischen Feld des äußeren Magnetes gelangt, es im Innenraum des Gehäuses hin- und herbewegbar und verdrehbar ist. Um die Funktionsweise zu erhalten, muß zunächst das Endoskop so verschwenkt werden, bis die äußeren und inneren Magnete wieder in Wirkverbindung stehen.

Eine ähnliche Konstruktion ist aus dem DE 88 10 044 U1 bekannt, bei dem ein an der Innenseite des äußeren Ringes angeordneter Magnet mit einem sehr langerstreckten inneren Magnet in Verbindung steht.

Der äußere Magnet wird axial bewegt und zieht damit den inneren Magnet mit und bewirkt dadurch die Kupplung.

Ein Fernrohr mit diesem Grundprinzip der Objektivverstellung ist auch aus der deutschen Patentschrift 970 298 bekannt. Auch hier ist möglich, daß bei mechanischen Schocks der innere Magnet außer magnetischem Eingriff mit dem äußeren Magnet kommt.

Es ist Aufgabe der vorliegenden Erfindung, eine Vorrichtung der eingangs genannten Art dahingehend zu verbessern, daß bei kleiner Bauweise eine effektive Positionierung von Bauelementen auf Dauer funktionssicher durchführbar ist.

Erfindungsgemäß wird die Aufgabe dadurch gelöst, daß sowohl das äußere als auch das innere Ringelement als drehbare, jedoch axial unverschieblich angeordnete Ringe ausgebildet sind, die gegenüberliegend die Magnete tragen, und daß der innere Ring mit den zu positionierenden Bauelementen mechanisch in Verbindung steht, wobei der innere Ring über einen Gewindegang mit einem axial im Gehäuse verschieblich angeordneten Bauelement in Verbindung steht, so daß eine Drehbewegung des inneren Ringes in eine Axialbewegung des Bauelementes umgesetzt wird.

Es wird nun von dem Prinzip abgewichen, daß zumindest einer oder gar beide Magnete axial beweglich sind.

Es sind nunmehr nur noch verdrehbare, die Magnete tragende Ringe vorhanden, so daß die Axialerstreckung der Magnetkupplung sehr kurz ist. Dadurch baut die Magnetkupplung nicht nur kurz, sondern es ist kein großflächiger axialer Bereich vorhanden, über den störende Magnetfelder abgestrahlt werden können, es kann somit effektiv die störende Strahlung, d.h. ohne große ausladende Baumaßnahmen, abgeschirmt werden.

Die Relativstellung zwischen innerem und äußerem Magnet ändert sich beim Verdrehen nicht. Wird der äußere Ring mit dem äußeren Magnet gedreht, dreht sich entsprechend der innere Ring mit dem inneren Magnet. Es fallen somit schraubenlinienförmige Führungen für bewegte Magnete völlig weg. Die Umsetzung der Drehbewegung des inneren Ringes wird über die mechanische Verbindung zwischen den positionierenden Bauelementen und dem Ring bewerkstelligt.

Insgesamt gesehen baut die mechanische Kupplung axial sehr kurz und kommt auch nur mit zwei prinzipiellen Bauelementen aus, nämlich äußerer Ring mit den äußeren Magneten und innerer Ring mit den inneren Magneten, somit sind wenig störanfällige Teile vorhanden, die auf Dauer funktionssicher sind. Mechanische Schocks oder Stöße können aufgrund der Tatsache, daß die Ringe axial unverschieblich sind, nicht zu einer Lösung des magnetischen Kraftschlusses in axialer Richtung führen. Die axiale schmale Bauweise ermöglicht auch durch einfache Baumaßnahmen, die magnetische Abschirmung zu bewerkstelligen. Auch in radialer Richtung baut die magnetische Kupplung nicht ausladend, da die beiden in etwa in einer Ebene angeordneten Ringe wenig ausladende Bauelemente bilden.

Dadurch, daß der innere Ring über einen Gewindegang mit einem axial im Gehäuse verschieblich angeordneten Bauelement in Verbindung steht, so daß eine Drehbewegung des inneren Rings in eine Axialbewegung des Bauelements umgesetzt wird, wird die Drehbewegung des inneren Ringes durch einfache mechanische Mittel in eine Axialbewegung des Bauelements umgesetzt.

In einer weiteren Ausgestaltung der Erfindung weist der äußere Ring außen eine ferromagnetische Abschirmung auf, um störende, nach außen gerichtete magnetische Streufelder abzuschirmen.

Diese Maßnahme hat den Vorteil, daß durch die äußere Abschirmung sichergestellt ist, daß von der magnetischen Kupplung keine störenden, nach außen gerichteten magnetischen Streufelder ausgehen.

In einer weiteren Ausgestaltung der Erfindung besteht der äußere Ring aus ferromagnetischem Material, an dessen Innenseite der zumindest eine Magnet angebracht ist.

Diese Maßnahme hat nun den Vorteil, daß die Abschirmung zugleich das Trägerelement bzw. den eigentlichen Ring ausmacht, an dessen Innenseite der zumindest eine Magnet angebracht ist.

Dies führt ebenfalls zu baulich einfachen, wenig ausladend bauenden Teilen.

In einer weiteren Ausgestaltung der Erfindung sind umfänglich verteilt eine Vielzahl an radial polarisierten Magneten an der Innenseite des äußeren Ringes angeordnet.

Durch die Anordnung von einer Vielzahl an radial polarisierten Magneten kann der Ring in axialer Richtung äußerst schmal ausgebildet werden, da durch die Vielzahl der Magnete der notwendige magnetische Kraftschluß mit dem inneren Magnet in jeder Drehstellung ausgebildet werden kann.

In einer weiteren Ausgestaltung der Erfindung ist der äußere Ring als Stellring ausgebildet.

Diese Maßnahme hat den Vorteil, daß der äußere Ring auch zugleich als Stellring fungiert, dieser also von der Außenseite ergriffen werden kann, somit besonders wenige Bauteile notwendig sind, was zu einer auch in radialer Richtung schlanken Bauweise führt.

In einer weiteren Ausgestaltung der Erfindung ist der äußere Ring außen mit einem Stellring verbunden.

Diese Maßnahme hat nun den Vorteil, daß das Material von äußerem Ring und diesen umgebenden Stellring frei ausgewählt werden kann, so daß auch Designansprüchen Rechnung getragen werden kann, wenn sich bspw. die äußere Gestaltung des Stellringes an die weitere äußere Ausgestaltung des endoskopischen Systems anpassen soll. Es ist auch möglich, den Stellring zugleich als Abschirmung auszubilden und den inneren eigentlichen Ring bspw. aus Kunststoff auszubilden, in den dann die Magnete eingegossen sind. Der äußere Ring kann manuell verdreht oder über einen motorischen, insbesondere elektromotorischen Antrieb bewegt werden.

In einer weiteren Ausgestaltung der Erfindung sind auf der äußeren Seite des inneren Ringes umfänglich verteilt eine Vielzahl an radial polarisierten Magneten angeordnet.

Diese Maßnahme hat den erheblichen Vorteil, insbesondere mit der zuvor erwähnten Maßnahme der Vielzahl an Magneten an der Innenseite des äußeren Ringes, daß mit extrem axial schmal bauenden Ringen ein ausreichender Kraftschluß geschaffen werden kann, um auch relativ schwere oder schwergängige Bauelemente zu bewegen. Dies trägt ferner zu einer wenig ausladenden, also sowohl in axialer als auch in radialer Richtung schlanken Bauweise bei.

In einer weiteren Ausgestaltung der Erfindung sind Anzahl, umfängliche Verteilung und axiale Ausdehnung der Magnete von innerem Ring und äußerem Ring gleich.

Diese Maßnahme hat den erheblichen Vorteil, daß sich zwischen den einzelnen identischen gegenüberstehenden Magneten ein intensiver magnetischer Fluß ausbildet, so daß auch Relativverschiebungen zwischen äußerem und innerem Ring bei ungeschicktem oder rückartigem Bewegen des äußeren Rings nicht stattfinden. Dies trägt erheblich zur Betriebssicherheit bei.

In einer weiteren Ausgestaltung der Erfindung ist das axial verschiebliche Bauelement gegen Verdrehen gesichert.

Diese Maßnahme hat insbesondere bei optischen Systemen den Vorteil, daß dieses ausschließlich axial bewegt wird und nicht ebenfalls verdreht wird, wodurch bei komplizierten optischen Systemen, insbesondere bei asphärischen Linsen, Bildverschiebungen auftreten könnten.

In einer weiteren Ausgestaltung der Erfindung ist das axial verschiebliche Bauelement in axialer Richtung durch die Kraft einer Feder beaufschlagt.

Diese Maßnahme hat nun den Vorteil, daß das zwingend vorhandene Spiel der mechanischen Umsetzung der Drehbewegung in die Axialbewegung durch die Kraft der Feder in einer ganz bestimmten Endstellung des Spieles sich einstellt und auch die Umkehr der axialen Verschiebebewegung ruckfrei und sanft gefedert erfolgt.

In einer weiteren Ausgestaltung der Erfindung ist der Verdrehbereich des äußeren Ringes begrenzt.

Diese an sich bekannte Maßnahme hat den Vorteil, daß kein Überdrehen möglich ist.

In einer weiteren Ausgestaltung der Erfindung sind die Magnete als Sm-Co-Magnete ausgeführt, welche vorzugsweise eine Legierung aus SmCo₅ und/oder Sm₂Co₁₇ auf Basis von Eisenmetall aufweisen.

Diese Maßnahme hat den Vorteil, daß solche Samarium-Cobalt-Magnete eine hohe Temperaturbeständigkeit bis 200°C aufweisen. Die extremen Temperaturschwankungen, denen endoskopische Systeme beim Autoklavieren ausgesetzt sind, beeinträchtigen die Magnetisierung nicht.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: einen Längsschnitt einer erfindungsgemäßen Vorrichtung zum axialen Positionieren von optischen Bauelementen,
- Fig. 2: einen Schnitt längs der Linie II-II in Fig. 1,
- Fig. 3: einen Schnitt längs der Linie III-III in Fig. 1, und

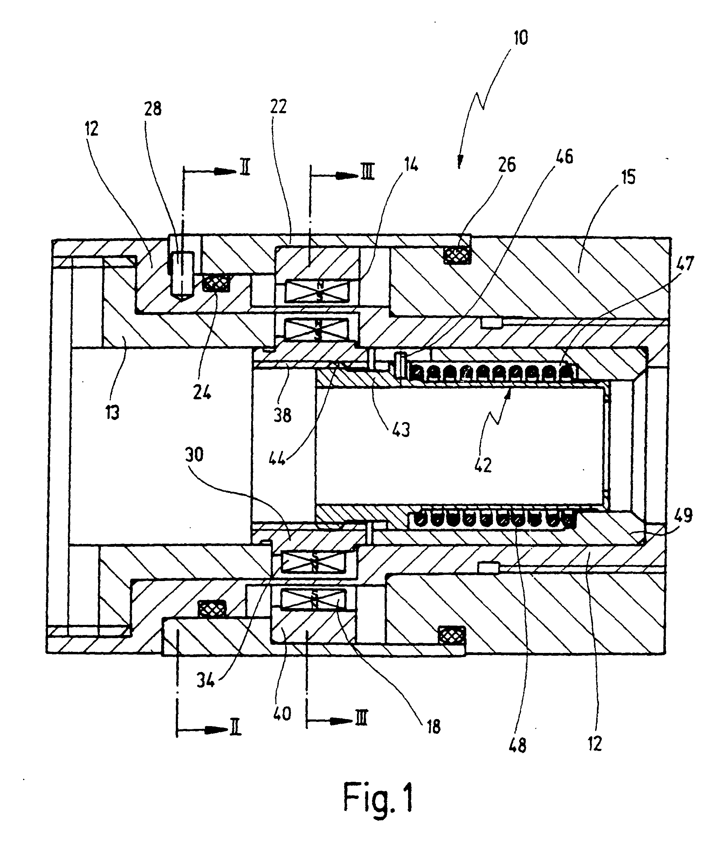

Eine in den Fig. 1 bis 3 dargestellte Vorrichtung ist in ihrer Gesamtheit mit der Bezugsziffer 10 bezeichnet.

Die Vorrichtung 10 ist Teil eines Optikkopfes eines Endoskopes, wobei sich in der Darstellung von Fig. 1 an der rechten Seite eine hier der Übersichtlichkeit nicht dargestellte Okularmuschel anschließt, an der linken Seite der Darstellung von Fig. 1 ist der Schaft des Endoskopes angebracht.

Die Vorrichtung 10 weist ein grob hohlzylindrisches Gehäuse 12 auf, das umfänglich aus einem Stück besteht, und somit hermetisch abgeschlossen ist.

An den Stirnseiten des Gehäuses 12 bilden die zuvor erwähnten Bauelemente, also Okularmuschel einerseits und Endoskopschaft andererseits, einen entsprechenden dichtenden Abschluß.

Eine innere Montagehülse 13 und eine äußere Montagehülse 15 dienen als Montageelemente für die im Gehäuse 12 aufgenommenen, nachfolgend noch zu beschreibenden Elemente.

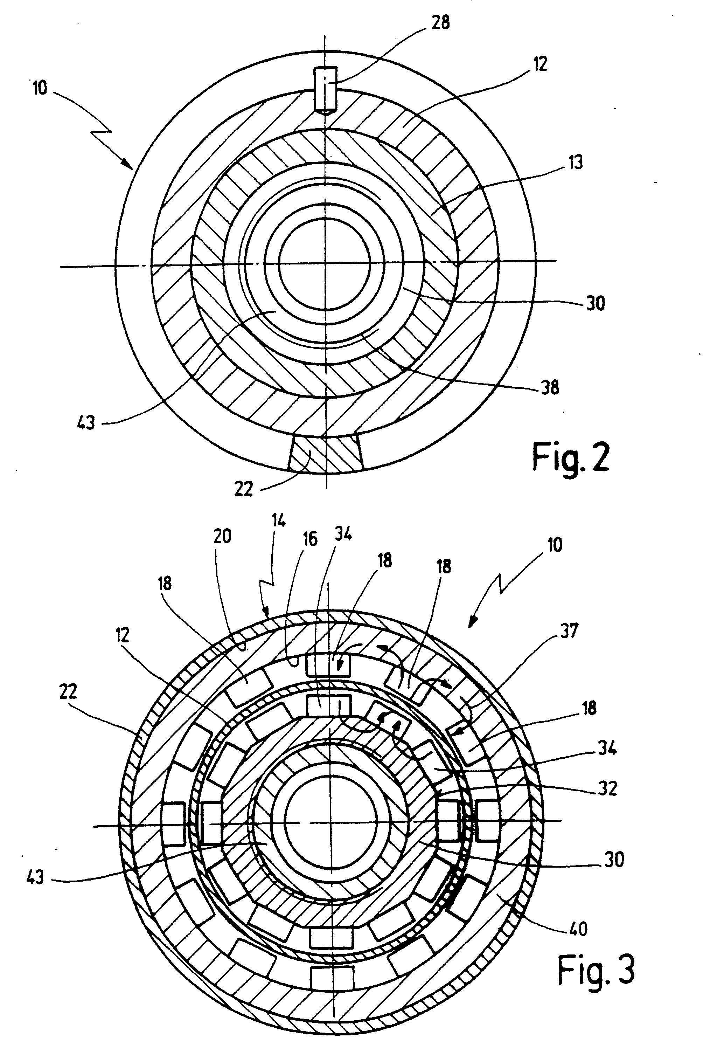

Über die Außenseite des Gehäuses 12 ist ein äußerer Ring 14 geschoben, dessen Innenseite 16 umfänglich gleichmäßig verteilt mit zwölf radial polarisierten Rundmagneten 18 versehen ist. Wie insbesondere aus der Schnittdarstellung von Fig. 3 zu erkennen, kommt die innere Seite der Magnete 18 in einem geringfügigen Abstand zur Außenseite des Gehäuses 12 zum Liegen.

Auf der Außenseite 20 des äußeren Ringes 14 ist ein Stellring 22 montiert, der axial wesentlich länger als der äußere Ring 14 ist und der über O-Ringdichtungen 24 und 26 dichtend, jedoch verdrehbar um die Außenseite des Gehäuses 12 angebracht ist. Ein radial vorspringender Anschlag 28 im Gehäuse 12 begrenzt die umfängliche Verdrehbarkeit des äußeren Ringes 14, wie das insbesondere aus Fig. 2 ersichtlich ist.

Im Innern des Gehäuses 12 ist ein innerer Ring 30 montiert, dessen äußere Seite 32 ebenfalls mit zwölf Rundmagneten 34 versehen ist.

Wie das insbesondere aus der Schnittdarstellung von Fig. 3 ersichtlich ist, ist die umfängliche Verteilung und Anordnung der Magnete 34 so, daß jeweils ein Magnet 34 direkt einem entsprechenden Magnet 18 gegenüberliegt. Die Außenseite der Magnete 34 erstreckt sich bis nahezu an die Innenseite des Gehäuses 12 heran.

Die Magnete sind somit nur in einem äußerst geringfügigen radialen Abstand zueinander angeordnet, jedoch durch das hermetisch abgedichtete Gehäuse 12 voneinander getrennt. Die Magnete 34 sind ebenfalls radial polarisiert und deren Polarität ist so ausgerichtet (siehe Fig. 1 und Fig. 3), daß ein Magnetfluß stattfindet, wie er in Fig. 3 durch die Linien 37 angedeutet ist. Die Magnete 18 und 34 sind als Sm-Co-Magnete ausgeführt.

In dem dargestellten Ausführungsbeispiel besteht der äußere Ring 14 aus einem ferromagnetischen Material, bildet somit eine Abschirmung 40 gegenüber der Außenseite. Das heißt, es treten über die Außenseite keine Magnetfelder hinaus. Das ferromagnetische Material bildet gegenüber der Außenseite, also der Luft, einen wesentlich geringeren magnetischen Widerstand, so daß nahezu die Gesamtheit des äußeren Magnetflusses über die Abschirmung 40 läuft.

Im Inneren des Gehäuses 12 ist ein Bauelement 42 angeordnet, das eine Hülse 43 aufweist. In der Hülse 43 sind optische Bauelemente wie bspw. Linsen angeordnet, die der Übersichtlichkeit halber nicht dargestellt sind.

Die Außenseite der Hülse 43 ist mit einer radial vorspringenden Nase 44 versehen, die in einen Gewindegang 38 an der Innenseite 36 des inneren Rings 30 eingreift.

Ein von der Außenseite 47 der Hülse 43 radial vorstehender Stift 46 dient als Verdrehsicherung, er greift in eine entsprechend hier nicht näher bezeichnete Axialnut in eine die Hülse 43 umgebende Außenhülse 49 ein.

In dem Raum zwischen Außenhülse 49 und Hülse 43 ist eine vorgespannte schraubenlinienförmige Feder 48 angeordnet, die die axial verschiebliche Hülse 43 gegenüber der ortsfesten Außenhülse 49 axial mit Druck beaufschlagt.

Wird nunmehr der äußere Ring 14 über den Stellring 22 verdreht, wird auch der innere Ring 30 aufgrund des Kraftschlusses zwischen den Magneten 18 und 34 verdreht. Diese Verdrehbewegung wird über den Gewindegang 28 und die Nase 44 in eine axiale Verschiebung der Hülse 43 umgesetzt.

Dadurch können die in der Hülse 43 aufgenommenen optischen Bauelemente gegenüber weiteren im Optikkopf aufgenommenen Bauelementen verschoben, bspw. justiert oder fokussiert werden.

## Patentansprüche

1. Vorrichtung zum Positionieren von Bauelementen (42) innerhalb endoskopischer Systeme, mit einem hermetisch dichten Gehäuse (12), mit einem um die Außenseite des Gehäuses (12) drehbar angeordneten äußeren Ringelement, das umfänglich zumindest einen Magneten (18) trägt, mit einem weiteren, innerhalb des dichten Gehäuses (12) angeordneten inneren Ringelement, das ebenfalls zumindest einen Magneten (34) trägt, wobei die Magnete (18, 34) so ausgerichtet sind, daß durch eine Drehbewegung des äußeren Ringelementes über magnetischen Kraftschluß der zumindest eine innere Magnet (34) bewegt wird, wobei diese Bewegung zum Positionieren von Bauelementen (42) dient, **dadurch gekennzeichnet, daß** sowohl das äußere als auch das innere Ringelement als drehbare, jedoch axial unverschieblich angeordnete Ringe (14, 30) ausgebildet sind, die auf den sich zugewandten Seiten gegenüberliegend die Magnete (18, 34) tragen, und daß der innere Ring (30) mit den zu positionierenden Bauelementen (42) mechanisch in Verbindung steht, wobei der innere Ring (30) über einen Gewindegang (38) mit einem axial im Gehäuse (12) verschieblich angeordneten Bauelement (42) in Verbindung steht, so daß eine Drehbewegung des inneren Ringes (30) in eine Axialbewegung des Bauelementes (42) umgesetzt wird.

2. Vorrichtung nach Anspruch 1, **dadurch gekennzeichnet, daß** der äußere Ring (14) außen eine ferromagnetische Abschirmung (40) aufweist, um störende, nach außen gerichtete magnetische Streufelder abzuschirmen.

3. Vorrichtung nach Anspruch 2, **dadurch gekennzeichnet, daß** der äußere Ring (14) aus ferromagnetischem Material besteht, an dessen Innenseite (16) der zumindest eine Magnet (18) angebracht ist.

4. Vorrichtung nach einem der Ansprüche 1 bis 3, **dadurch gekennzeichnet, daß** umfänglich verteilt eine Vielzahl an radial polarisierten Magneten (18) an der Innenseite (16) des äußeren Ringes (14) angeordnet ist.

5. Vorrichtung nach einem der Ansprüche 1 bis 4, **dadurch gekennzeichnet, daß** der äußere Ring (14) als Stellring ausgebildet ist.

6. Vorrichtung nach einem der Ansprüche 1 bis 5, **dadurch gekennzeichnet, daß** der äußere Ring (14) außen mit einem Stellring (22) verbunden ist.

7. Vorrichtung nach einem der Ansprüche 1 bis 6, **dadurch gekennzeichnet, daß** auf der äußeren Seite (32) des inneren Ringes (30) umfänglich verteilt eine Vielzahl an radial polarisierten Magneten (34) angeordnet ist.

8. Vorrichtung nach Anspruch 7, **dadurch gekennzeichnet, daß** Anzahl, umfängliche Verteilung und axiale Ausdehnung der Magnete (18, 34) von innerem Ring (30) und äußerem Ring (14) gleich sind.

9. Vorrichtung nach einem der Ansprüche 1 bis 8, **dadurch gekennzeichnet, daß** das axial verschiebliche Bauelement (42) gegen Verdrehen gesichert ist.

10. Vorrichtung nach einem der Ansprüche 1 bis 9, **dadurch gekennzeichnet, daß** das axial verschiebliche Bauelement (42) in axialer Richtung durch die Kraft einer Feder (48) beaufschlagt ist.

11. Vorrichtung nach einem der Ansprüche 1 bis 10, **dadurch gekennzeichnet, daß** der Verdrehbereich des äußeren Ringes (14) begrenzt ist.

12. Vorrichtung nach einem der Ansprüche 1 bis 11, **dadurch gekennzeichnet, daß** die Magnete (18, 34) als Sm-Co-Magnete ausgeführt sind, welche vorzugsweise eine Legierung aus SmCo₅ und/oder Sm₂Co₁₇ auf Basis von Eisenmetall aufweisen.

## Claims

1. A device for positioning components (42) within endoscopic systems, comprising a hermetically sealed housing (12), an outer ring element mounted to rotate about the outside of the housing (12) and carrying on its circumference at least one magnet (18), another inner ring element mounted inside the sealed housing (12) and likewise carrying at least one magnet (34), the magnets (18, 34) being aligned in such a way that any rotation of the outer ring element has the effect to move the at least one inner magnet (34) by magnetic coupling, such movement serves for positioning of components (42), **characterized in that** both, the outer and the inner ring elements, are configured as rotatable, but axially non displaceable rings (14, 30) carrying said oppositely arranged magnets (18, 34) on sides facing each other, and that the inner ring (30) is mechanically connected with the components (42) to be positioned, wherein the inner ring (30) is connected, via a thread (38), with a component (42) arranged for axial displacement in said housing (12) so that a rotary movement of said inner ring (30) will be translated into an axial movement of said component (42).

2. The device of claim 1, **characterized in that** said outer ring (14) is provided on its outside with a ferromagnetic screening (40) in order to screen any interfering, outwardly directed magnetic stray fields.

3. The device of claim 2, **characterized in that** said outer ring (14) is made from a ferromagnetic material with said at least one magnet (18) mounted on its inside (16).

4. The device of anyone of claims 1 through 3, **characterized in that** a plurality of radially polarized magnets (18) are mounted on the inside (16) of said outer ring (14) in circumferentially distributed arrangement.

5. The device of anyone of claims 1 through 4, **characterized in that** said outer ring (14) is configured as a set collar.

6. The device of anyone of claims 1 through 5, **characterized in that** said outer ring (14) is connected on its outside with a set collar (22).

7. The device of anyone of claims 1 through 6, **characterized in that** a plurality of radially polarized magnets (34) are provided on the outside (32) of said inner ring (30) in circumferentially distributed arrangement.

8. The device of claim 7, **characterized in that** the number, circumferential distribution and axial extension of the magnets (18, 34) are equal for said inner ring (30) and said outer ring (14).

9. The device of anyone of claims 1 through 8, **characterized in that** said axially movable component (42) is protected against rotation.

10. The device of anyone of claims 1 through 9, **characterized in that** said axially displaceable component (42) is subjected to the force of a spring (48) in the axial direction.

11. The device of anyone of claims 1 through 10, **characterized in that** the range of rotation of said outer ring (14) is limited.

12. The device of anyone of claims 1 through 11, **characterized in that** the magnets (18, 34) are designed as SmCo magnets, comprising preferably an alloy of SmCo₅ and/or Sm₂Co₁₇ based on ferrous metal.

## Revendications

1. Dispositif pour positionner des composants (42) à l'intérieur de systèmes endoscopiques, comportant un boîtier (12) hermétiquement étanche, comportant un élément annulaire extérieur monté tournant sur le côté extérieur du boîtier (12) et qui porte périphériquement au moins un aimant (18), comportant un autre élément annulaire intérieur monté à l'intérieur du boîtier (12) étanche, qui porte également au moins un aimant (34), les aimants (18, 34) étant orientés de manière que par un mouvement de rotation de l'élément annulaire extérieur, le ou les aimants intérieurs (34) soient déplacés par une force magnétique, ce mouvement servant à positionner des composants (42), **caractérisé en ce que** l'élément annulaire extérieur comme l'élément annulaire intérieur sont conformés en anneaux (14, 30) tournants, mais disposés de manière à ne pouvoir coulisser axialement, lesquels portent en vis-à-vis, sur leurs côtés tournés l'un vers l'autre, les aimants (18, 34), et **en ce que** l'anneau intérieur (30) est en liaison mécanique avec les composants (42) à positionner, l'anneau intérieur (30) étant en liaison, par un filetage (38), avec un composant (42) monté coulissant axialement dans le boîtier (12), de sorte qu'un mouvement de rotation de l'anneau intérieur (30) est converti en un mouvement axial du composant (42).

2. Dispositif selon la revendication 1, **caractérisé en ce que** l'anneau extérieur (14) comporte à l'extérieur un blindage ferromagnétique (40) afin d'assurer un blindage contre des champs de dispersion magnétiques perturbateurs, dirigés vers l'extérieur.

3. Dispositif selon la revendication 2, **caractérisé en ce que** l'anneau extérieur (14) est constitué d'une matière ferromagnétique, sur la face intérieure (16) de laquelle est fixé le ou les aimants (18).

4. Dispositif selon l'une des revendications 1 à 3, **caractérisé en ce qu'**un grand nombre d'aimants (18) polarisés radialement sont disposés, répartis périphériquement, sur la face intérieure (16) de l'anneau extérieur (14).

5. Dispositif selon l'une des revendications 1 à 4, **caractérisé en ce que** l'anneau extérieur (14) est conformé en anneau de réglage.

6. Dispositif selon l'une des revendications 1 à 5, **caractérisé en ce que** l'anneau extérieur (14) est relié extérieurement à un anneau de réglage (22).

7. Dispositif selon l'une des revendications 1 à 6, **caractérisé en ce que** sur la face extérieure (32) de l'anneau intérieur (30), sont disposés, répartis périphériquement, un grand nombre d'aimants (34) polarisés radialement.

8. Dispositif selon la revendication 7, **caractérisé en ce que** le nombre, la distribution périphérique et l'étendue axiale des aimants (18, 34) de l'anneau intérieur (30) et de l'anneau extérieur (14) sont identiques.

9. Dispositif selon l'une des revendications 1 à 8, **caractérisé en ce que** le composant (42) coulissant axialement est bloqué en rotation.

10. Dispositif selon l'une des revendications 1 à 9, **caractérisé en ce que** le composant (42) coulissant axialement est sollicité dans la direction axiale par la force d'un ressort (48).

11. Dispositif selon l'une des revendications 1 à 10, **caractérisé en ce que** la plage de rotation de l'anneau extérieur (14) est limitée.

12. Dispositif selon l'une des revendications 1 à 11, **caractérisé en ce que** les aimants (18, 34) sont réalisés sous la forme d'aimants SmCo qui présentent de préférence un alliage SmCo₅ et/ou Sm₂Co₁₇ à base d'un métal ferreux.
